# EUROPÄISCHE PATENTANMELDUNG

(11) **EP 0 656 208 A2**
(43) Veröffentlichungstag der Anmeldung: **07.06.1995**
(21) Anmeldenummer: 94113498.3
(22) Anmeldetag: 30.08.1994
(51) Int. Cl.: A61K 31/35, A61K 35/10

(54) **Verwendung von synthetischen Huminaten zur Herstellung eines Arzneimittels zur Behandlung von Schleimhauterkrankungen**

(30) Priorität: 27.11.1993 DE 4340484; 13.04.1994 DE 4412613; 13.04.1994 DE 4412612
(71) Anmelder: RÜTGERSWERKE AKTIENGESELLSCHAFT, 60326 Frankfurt (DE)
(72) Erfinder: Kühnert, Manfred, Prof., D-04299 Leipzig (DE); Haase, Anton F., Dr., D-64367 Mühltal (DE); Kleffner, Hans Werner, Dr., D-67271 Battenberg (DE)

(57) **Zusammenfassung**

Synthetische, durch Oxidation mehrwertiger phenolischer Verbindungen hergestellte Huminate dienen zur Herstellung von Mitteln mit Wirksamkeit im Schleimhautbereich.

Diese Mittel finden im human- und tiermedizinischen Bereich Verwendung und zwar bevorzugt im Bereich der Magen- und Darmschleimhaut, im Respirationstrakt und im Genitalbereich.

## Beschreibung

Die Erfindung betrifft eine neue Verwendung von synthetischen Huminaten zur Herstellung pharmazeutisch wirksamer Mittel.

Entsprechende Huminate sind bekannt aus EP-A 0 281 678, EP-A 0 537 427 oder EP-A 0 537 429.

Es sind dies synthetisch durch Oxidation und dadurch bewirkte Polykondensation von mehrwertigen Phenolen in schwach alkalischem wäßrigem Medium entsprechend EP-A 0 281 678 hergestellte niedermolekulare Ammonium- oder Alkalihuminate. Es sind dunkelbraune, wasserlösliche Produkte mit einem mittleren Molekulargewicht von 1 000, bei einem Streubereich von 250 bis 1 500.

Ihre wäßrigen Lösungen zeigen keinen Tyndall-Effekt und fluoreszieren nicht.

Weiterhin erfindungsgemäß einsetzbar sind aber auch synthetische Huminate mit Molekulargewichten bis zu 50 000 D, sofern sie den folgenden Merkmalen entsprechen:
Sie sind physiologisch aktiv, d.h., sie haben heilende Eigenschaften, sind sehr gering toxisch und weder mutagen, embryotoxisch noch teratogen, bzw. auch nicht cancerogen.

Ihre wäßrigen Lösungen zeigen keinen Tyndall-Effekt und fluoreszieren nicht. Entsprechende Prüfungen dieser Eigenschaften werden zweckmäßigerweise mit wäßrigen Lösungen in einer Konzentration durchgeführt, daß sie noch etwa 50 % der Transmission des eingestrahlten Lichtes haben. Beispiele für entsprechende, synthetische Huminate sind die gemäß EP-A 0 537 427 hergestellten Produkte.

Ebenso erfindungsgemäß verwendbar sind modifizierte Ammonium- oder Alkalihuminate, wie sie aus EP-A 0 537 429 bekannt sind. Sie werden hergestellt durch Oxidation der Verbindungen der allgemeinen Formeln 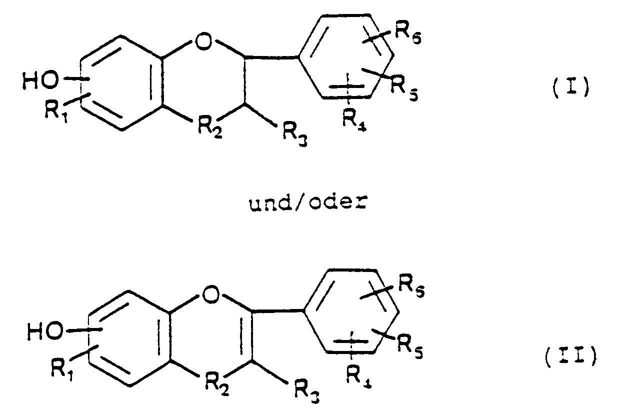
wobei R₁, R₃ und R₄ gleich oder verschieden sind und eine OH-Gruppe oder Wasserstoff darstellen, R₂ eine CO- oder CH2-Gruppe bedeutet und R₅ und R₆ gleich oder verschieden sind und Wasserstoff, eine OH- oder Methoxygruppe darstellen, wobei R₄, R₅ und R₆ nicht alle gleichzeitig Wasserstoff sind, in alkalischer wäßriger Lösung (pH 9).

Dabei können die Verbindungen gemäß der Formeln I und II als einzelne Reinsubstanzen sowie auch im beliebigen Gemisch miteinander eingesetzt werden. Sie können im Reaktionsgemisch auch mit mehrwertigen Phenolen eingesetzt werden, was aus wirtschaftlichen Gründen durchaus angebracht sein kann.

Es wurde gefunden, daß diese Huminate bei Irritationen und Erkrankungen von Schleimhaut bei Menschen und Tieren eine entzündungshemmende und heilende Wirkung haben. Entzündungen klingen nach Huminatbehandlung sehr schnell ab.

Entsprechende Schleimhautbereiche sind insbesondere die des Magens, Darms, der Genitalien und des Respirationstraktes. Dabei sind bei den einzelnen Schleimhautbereichen folgende Besonderheiten:

Es wurde gefunden, daß die synthetischen, relativ niedermolekularen, wasserlöslichen Alkali- oder Ammoniumhuminate im Magen- und Darmbereich eine entzündungshemmende und entgiftende Wirkung haben. Im Gegensatz zu den bislang in Fütterungsarzneimitteln bzw. als Nahrungs- bzw. Futterzusatzstoffen eingesetzten Huminaten werden sie jedoch im Bereich der Darmschleimhaut absorbiert und eingelagert und bewirken dort über einen verlängerten Zeitraum eine etwa gleichbleibende Wirkung, auch wenn der sie enthaltende Nahrungsbrei den Darm längst passiert hat, d.h., diese erfindungsgemäß eingesetzten Huminate zeigen einen deutlichen Retard-Effekt.

Im Schleimhautbereich der Genitalien von Menschen und Tieren zeigen die synthetischen Huminate eine entzündungshemmende und heilende Wirkung. Die Huminate werden an den Schleimhäuten absorbiert und wirken somit lokal, wobei sie über einen längeren Zeitraum (6-8 h) eine etwa gleichbleibende Wirkung zeigen. Die normale Mikroorganismenflora wird nicht geschädigt, jedoch wird eine Überflutung der behandelten Bereiche mit schädlichen Keimen zurückgedrängt. Insbesondere katarrhalische Entzündungen klingen nach Huminatbehandlung sehr schnell ab.

Auch im Schleimhautbereich des Respirationstrakts von Menschen und Tieren wird eine entzündungshemmende und heilende Wirkung der synthetischen Huminate beobachtet. Auch hier werden die Huminate an den Schleimhäuten absorbiert und wirken somit lokal, wobei sie über einen längeren Zeitraum (6-8 h) eine etwa gleichbleibende Wirkung zeigen. Sie wirken dabei sofort reizdämpfend, bilden einen Film auf der Schleimhaut und bewirken eine rasche Ruhigstellung des Erkrankten. Katarrhalische Entzündungen klingen nach Huminatbehandlung sehr schnell ab.

Die synthetisch durch Oxidation mehrwertiger phenolischer Verbindungen hergestellten Huminate eignen sich daher zur Herstellung von Mitteln mit Wirksamkeit im Schleimhautbereich, wie in Anspruch 1 beansprucht, insbesondere mit Wirksamkeit im Bereich der Magen- und Darmschleimhaut gemäß Anspruch 2, im Respirationstrakt gemäß Anspruch 3 und im Genitalbereich gemäß Anspruch 4.

Diese Mittel finden sowohl im human- als auch im tiermedizinischen Bereich Verwendung, wie in Anspruch 5 beansprucht. Die Mittel mit Wirksamkeit im Bereich der Magen- und Darmschleimhaut finden als Fütterungsarzneimittel oder als Futtermittelzusatzstoffe Verwendung, wie in den Ansprüchen 6 und 7 beansprucht. Die Mittel mit Wirksamkeit im Respirationstrakt finden vorwiegend Verwendung bei katarrhalischen Erkrankungen der Atmungswege auf infektiöser und nicht infektiöser Grundlage, bei Pseudokrupp-Husten und bei Raucherhusten entsprechend der Ansprüche 8-10.

Die Mittel mit Wirksamkeit im Genitalbereich finden vorwiegend Verwendung im Vaginal-, Uteral- und Präputialbereich sowie zur Behandlung von aufsteigenden Harnröhrenentzündungen sowie zur lokalen Tumorbehandlung im Genitalbereich entsprechend der Ansprüche 11-15.

Es ist bekannt, daß natürliche Huminstoffe im Darmbereich eine entzündungshemmende und entgiftende Wirkung haben. Unter den Bezeichnungen Dystikum, Sulumin und Humocarb werden daher Tierarznei- und Fütterungsarzneimittel vertrieben, die ein Gemisch verschiedener natürlicher Huminate mit Molekulargewichten bis zu 100 000 als wirksame Komponente enthalten. Diese Mittel sind bei oraler Aufnahme nicht toxisch und zeigen auch nach lang andauernder Verabreichung keine unerwünschten Nebenwirkungen.

Desweiteren entstehen keine toxischen Abbauprodukte und auch die wieder ausgeschiedenen Mittel sind als natürliche Produkte anzusehen, die keine Belastung der Umwelt darstellen.

Es ist aber ein gravierender Nachteil dieser Huminstoffe, daß die Wirkung nur ein Passageeffekt ist, d. h., daß sie nur bei langer regelmäßiger und konstanter Verabreichung wirken.

Es bestand daher die Aufgabe, Mittel mit mindestens gleich guter Wirksamkeit im Magen- und Darmbereich wie die bekannten natürlichen Huminstoffe bereitzustellen, die auch hinsichtlich ihrer Toxizität, Nebenwirkungen und Umweltbelastung den natürlichen Huminstoffen entsprechen, deren Wirksamkeitsdauer aber wesentlich verlängert ist, so daß sowohl während ihrer Zufuhr aber auch später im Magen-Darm-Trakt eine entsprechende pharmakologische Wirksamkeit vorhanden ist.

Die Lösung der Aufgabe erfolgt durch die Verwendung von synthetisch durch Oxidation mehrwertiger phenolischer Verbindungen hergestellten, wasserlöslichen Alkali- oder Ammoniumhuminate mit einem Molekulargewicht bis zu 50 000 D als Human- und Tierarzneimittel sowie als Fütterungsarzneimittel bzw. zur Herstellung von oral aufzunehmenden Mitteln mit Wirksamkeit im Bereich der Magen- und Darmschleimhaut.

Es wurde gefunden, daß diese synthetischen, relativ niedermolekularen, wasserlöslichen Alkali- oder Ammoniumhuminate bei oraler Verabreichung ebenfalls im Magen- und Darmbereich eine entzündungshemmende und entgiftende Wirkung haben. Im Gegensatz zu den bislang in Fütterungsarzneimitteln bzw. als Nahrungs- bzw. Futterzusatzstoffen eingesetzten Huminaten werden sie jedoch im Bereich der Darmschleimhaut absorbiert und eingelagert und bewirken dort über einen verlängerten Zeitraum eine etwa gleichbleibende Wirkung, auch wenn der sie enthaltende Nahrungsbrei den Darm längst passiert hat, d. h., diese erfindungsgemäß eingesetzten Huminate zeigen einen deutlichen Retard-Effekt.

Entzündete Magen- und Darmschleimhaut schwillt schneller ab. Viren und bakterielle Keime werden verstärkt inaktiviert. Dadurch kann die zur gewünschten Wirkung verabreichte Dosis reduziert werden.

Obwohl die Huminate an der Darmschleimhaut absorbiert werden, werden sie nicht vom Körper aufgenommen. D. h. einerseits, daß diese Huminate lokal wirken. Andererseits aber bedeutet dies auch, daß Tiere bis zur Schlachtung mit diesen Huminaten oral behandelt werden können, ohne daß die Gefahr besteht, daß Spuren der Huminate oder ihrer Abbauprodukte in das Fleisch gelangen (keine Rückstandsbildungsprobleme).

Darüberhinaus aber zeigen die erfindungsgemäß eingesetzten Huminate überraschenderweise einen starken vermifugen Effekt. Nach etwa achttägiger regelmäßiger Verabreichung dieser Huminate sind die behandelten Tiere durchweg wurmfrei.

Diese Huminate eignen sich daher zur Verwendung als Nahrungs- oder Futtermittelzusatzstoffe, zur Herstellung von oral aufzunehmenden Arzneimitteln mit Wirksamkeit im Bereich der Magen- und Darmschleimhaut zur Verwendung in der Human- und Veterinärmedizin, sowie zur Herstellung von Fütterungsarzneimitteln.

Ein weiterer praktischer Vorteil der erfindungsgemäß eingesetzten Huminate besteht darin, daß sie vollständig wasserlöslich sind und somit in anderer Zubereitung eingesetzt werden können als die bislang verwendeten Huminstoffe, die immer in Kombination mit Zusatzstoffen formuliert werden mußten.

Die erfindungsgemäß eingesetzten Huminate können im einfachsten Fall dem Trinkwasser der Tiere zugesetzt werden oder eine wäßrige Lösung der Huminate kann auf das Futter aufgesprüht oder mit ihm vermischt werden.

Bevorzugt jedoch wird ein Fütterungsarzneimittel oder ein Futtermittelzusatzstoff hergestellt, der aus 1 bis 60 Gew.-% Huminat und 99 bis 40 Gew.-% eines Füllstoffes besteht. Derartige Füllstoffe können entweder inerte anorganische Materialien, wie z. B. Silikate oder Schwerspat sein oder natürliches organisches Material, insbesondere cellulosehaltiges Material, wie z. B. Weizennachmehl, Johannisbrotkernmehl oder gemahlene Spelzen.

Die Dosierung der erfindungsgemäß eingesetzten Huminate soll so erfolgen, daß pro kg Körpergewicht pro Tag eine Menge von 0,05 bis 5 g Huminat verabreicht werden.

Die Mittel mit Wirksamkeit im Respirationstrakt sind bevorzugt versprühbare Zubereitungen. Zusätzlich zur heilenden und entzündungshemmenden Wirkung haben die Huminate bei diesem Verwendungszweck weitere Vorteile: Sie sind nicht toxisch, sondern eine Vielzahl von Toxinen werden von ihnen gebunden und somit als störende Komponenten aus dem behandelten Bereich entfernt. Durch die Absorption der Huminate an der Schleimhaut ohne Bioresorbierbarkeit werden hohe Verweilzeiten und eine gute Bedeckung der Schleimhaut erreicht. Die Behandlung muß nicht oft wiederholt werden. Im Gegensatz zu Chemotherapeutika erfolgt keine Transformation durch die Gewebe und somit auch keine Rückstandsbildung. Andererseits wird eine beruhigende und antispastische Wirkung beobachtet. Reizhustenanfälle werden gedämpft.

Die Wirkung der Huminate ist als viruzid anzusehen, nicht jedoch als generell antibakteriell. D.h., das biologische Gleichgewicht auf der behandelten Schleimhaut bleibt erhalten.

Vor allem aber besteht ein wesentlicher Vorteil bei der Behandlung des Respirationstrakts mit synthetischen Huminaten darin, daß die Zilien des Respirationstrakts nicht geschädigt werden.

Die Huminat enthaltenden Mittel eignen sich somit insbesondere zur Behandlung von Katarrhen und entzündlichen Veränderungen der Schleimhaut im Respirationstrakt.

Die Mittel sind im einfachsten Falle bis zu 10 %ige, bevorzugt 0,1-5 %ige wäßrige Lösungen der Huminate, die als Sprühnebel bzw. als Inhalationssprays zur trachealen, bronchialen oder alveolaren Behandlung bei hyperkinetischen Schleimhautsituationen mit exogener oder endogener Ursache verwendet werden.

So wurden z.B. Pferden, die am sog. Virushusten erkrankt waren, 1 %ige wäßrige Lösungen von Huminaten, hergestellt durch Oxidation von Hydrochinon gemäß dem Beispiel der EP-B 0 281 678, während des Einatmens in den Rachenraum gesprüht. Diese Behandlung wurde jeweils täglich einmal durchgeführt. Nach 4- bis 6-maliger Behandlung waren die Pferde beschwerdefrei.

Hustende Sportpferde wurden in analoger Weise mit 0,1 %iger, wäßriger Huminatlösung behandelt. Sie waren nach 2 Stunden frei von Hustenreiz und konnten in Rennen eingesetzt werden.

Zusätze von ätherischen Ölen oder spezifischen Arzneiwirkstoffen zu den Huminatlösungen sind möglich und manchmal auch wünschenswert.

Die Mittel können aber auch feste, feinvermahlene Huminate sein, die z.B. durch Ultraschallvernebelung festversprüht oder aerosolisiert werden. Durch die leichte Löslichkeit in Wasser bildet sich auf der Schleimhaut sofort ein Film mit gelösten Huminaten.

Mittel mit Wirksamkeit im Genitalbereich auf Huminatbasis sind entweder wäßrige Lösungen oder Gele sind oder Kapseln oder Stäbe, die die Huminate in Form wäßriger Lösungen, Gele oder Salben oder in fester Form als Reinsubstanz oder an ein Trägermaterial gebunden (vergl. EP-B 0 283 573) enthalten.

Zusätzlich zur heilenden und entzündungshemmenden Wirkung haben die Huminate bei diesem Vervendungszweck weitere Vorteile: Sie sind nicht toxisch, sondern eine Vielzahl von Toxinen werden von ihnen gebunden und somit als störende Komponenten aus dem behandelten Bereich entfernt. Durch die Absorption der Huminate an der Schleimhaut ohne Bioresorbierbarkeit werden hohe Verweilzeiten und eine gute Bedeckung der Schleimhaut erreicht. Die Behandlung muß nicht oft wiederholt werden. Im Gegensatz zu Chemotherapeutika erfolgt keine Rückstandsbildung. Andererseits wird eine beruhigende und antispastische Wirkung beobachtet.

Die Wirkung der Huminate ist als viruzid anzusehen, nicht jedoch als generell antibakteriell. D.h., das biologische Gleichgewicht auf der behandelten Schleimhaut bleibt erhalten.

Die Huminat enthaltenden Mittel eignen sich somit insbesondere zur Behandlung von Katarrhen und entzündlichen Veränderungen der Schleimhaut im Genitalbereich und zwar sowohl im peri- als auch im postpartalen Zustand.

Die Mittel sind im einfachsten Falle bis zu 10 %ige, bevorzugt 1-5 %ige wäßrige Lösungen der Huminate, die insbesondere zu Vaginal- und/oder Uteralspülungen eingesetzt werden.

Gele oder Salben enthalten bis zu 30 % Huminate sowie übliche Gelierungsmittel oder Salbengrundlage. Bevorzugt sind Hydrogele mit Gelierungsmitteln auf Basis Gelatine, Hydroxyalkylcellulose und/oder Polyoxyethylenen. Kapseln oder Stäbe, die bevorzugt in den Uteralbereich eingebracht werden, enthalten entweder konzentrierte Lösungen (bis zu 40 %ig) oder feste Huminate zusammen mit physiologisch verträglichen Füllstoffen und einem sog. Sprengmittel (z.B. 0,5-2 % Hydrogencarbonat).

### Beispiele

Das eingesetzte Huminat ist ein Na-Huminat mit einem durchschnittlichen Molekulargewicht von 1000 bei einem Streubereich von 300 bis 1500, hergestellt durch Oxidation von Hydrochinon gemäß dem Beispiel der EP-B 0 281 678.

### Beispiel 1

40 Milchkühe erhielten jeweils nach einer Schwergeburt im Abstand von 12 h 4 Uteralspülungen mit 2 l 4 %iger Huminatlösung.

Im Vergleich zu anderen Kühen wurde eine beschleunigte Regeneration des Uterus beobachtet. Die Kühe gaben nach 2 d bereits lebensmittelhygienisch unbedenklich Milch.

### Beispiel 2

Katarrhalische Erkrankungen der Vagina kleiner Haus- und Heimtiere (Hunde, Katzen) wurden täglich mit einem wäßrigen Gel aus 20 %iger Huminsäurelösung und 2 % Hydroxymethylcellulose behandelt. Ohne weitere Zusatzbehandlung waren die Katarrhe innerhalb von 7 Tagen ausgeheilt.

## Patentansprüche

1. Verwendung von synthetischen, durch Oxidation mehrwertiger phenolischer Verbindungen hergestellten Huminaten zur Herstellung von Mitteln mit Wirksamkeit im Schleimhautbereich.

2. Verwendung von synthetisch durch Oxidation mehrwertiger phenolischer Verbindungen hergestellten Huminaten zur Herstellung von oral aufzunehmenden Mitteln mit Wirksamkeit im Bereich der Magen- und Darmschleimhaut.

3. Verwendung von synthetischen, durch Oxidation mehrwertiger phenolischer Verbindungen hergestellten Huminaten zur Herstellung von Mitteln mit Wirksamkeit im Respirationstrakt.

4. Verwendung von synthetischen, durch Oxidation mehrwertiger phenolischer Verbindungen hergestellten Huminaten zur Herstellung von Mitteln mit Wirksamkeit im Genitalbereich.

5. Verwendung der Mittel gemäß der Ansprüche 1-4 im human- und tiermedizinischen Bereich.

6. Verwendung der Mittel gemäß der Ansprüche 1, 2 und 5 als Fütterungsarzneimittel.

7. Verwendung der Mittel gemäß der Ansprüche 1, 2 und 5 als Futtermittelzusatzstoffe.

8. Verwendung der Mittel gemäß der Ansprüche 1, 3 und 5 bei katarrhalischen Erkrankungen der Atmungswege auf infektiöser und nichtinfektiöser Grundlage.

9. Verwendung der Mittel gemäß der Ansprüche 1, 3 und 5 bei Pseudekrupp-Husten.

10. Verwendung der Mittel gemäß der Ansprüche 1, 3 und 5 bei Raucherhusten.

11. Verwendung der Mittel gemäß der Ansprüche 1, 4 und 5 im Vaginalbereich.

12. Verwendung der Mittel gemäß der Ansprüche 1, 4 und 5 im Uteralbereich.

13. Verwendung der Mittel gemäß der Ansprüche 1, 4 und 5 im Präputialbereich.

14. Verwendung der Mittel gemäß der Ansprüche 1, 4 und 5 zur Behandlung von aufsteigenden Harnröhrenentzündungen.

15. Verwendung der Mittel gemäß der Ansprüche 1, 4 und 5 zur lokalen Tumorbehandlung im Genitalbereich.
